(19) Europäisches Patentamt
European Patent Office
Office européen des brevets

(11) Publication number: **0 033 789**
**B1**

(12) # EUROPEAN PATENT SPECIFICATION

(45) Date of publication of patent specification: **07.03.84**

(51) Int. Cl.³: **C 07 C 91/32** //C07C93/14

(21) Application number: **80300272.4**

(22) Date of filing: **30.01.80**

(54) **2-alpha-Methyl-dopaminimino-6,7-dihydroxy-1,2,3,4-tetrahydronaphthalene, its salts, and a process for preparation thereof.**

(43) Date of publication of application:
**19.08.81 Bulletin 81/33**

(45) Publication of the grant of the patent:
**07.03.84 Bulletin 84/10**

(84) Designated Contracting States:
**AT BE CH IT LU NL SE**

(56) References cited:
**DE - C - 745 314**
**FR - A - 2 373 513**
**US - A - 4 010 202**

**CHEM. PHARM. BULL., vol. 25, no. 11, 1977, pages 2917-2928 KATSUMI ITOH et al.: "The syntheses and beta-adrenoceptor activities of N-substituted 2-amino-5,6-dihydroxy-1,2,3,4-tetrahydro-1-naphthalenols"**
**JOURNAL OF MEDICINAL CHEMISTRY, vol. 20, no. 9, September 1977, pages 1111-1116 Washington D.C., U.S.A. J.G. CANNON et al.: "Cerebral dopamine agonist properties of some 2-aminotetralin derivatives after peripheral and intracerebral administration"**

(73) Proprietor: **AMERICAN HOSPITAL SUPPLY CORPORATION**
**One American Plaza**
**Evanston, IL 60201 (US)**

(72) Inventor: **Stout, David Michael**
**18 Warrington Road**
**Vernon Hills Illinois 60061 (US)**

(74) Representative: **Seaborn, George Stephen**
**c/o Edward Evans & Co. Chancery House 53-64**
**Chancery Lane**
**London WC2A 1SD (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**EP 0 033 789 B1**

2-$\alpha$-methyl-dopaminimino-6,7-dihydroxy-1,2,3,4-tetrahydronaphthalene its salts, and a process for preparation thereof

This invention relates to derivatives of 2-amino-6,7-dihydroxy-tetrahydronaphthalene (ADTN).

FR—A—2 373 513 (SANDOZ SA) discloses 2-amino-tetrahydronaphthalenes of a general formula within which fall the compounds of the present invention. The compounds of FR—A—2 373 513 are known to exhibit inotropic potency (i.e. cause increased cardiac contractile force).

It is also known from Proc, Natl. Acad. Sci. U.S.A., Vol. 74 (1977), pages 3602—3606 (VOLKMAN et al) and JOURNAL OF MEDICINAL CHEMISTRY, Vol. 20 (1977) pages 1111—1116 (J.G. CANNON et al) that 2-amino-6,7-dihydroxytetrahydronaphthalene (6,7-ADTN) and derivatives thereof exhibit dopamine-like behaviour. In particular it is known from the former publication that 6,7-ADTN and N-methyl 6,7-ADTN act to cause vasodilation in an analogous manner to dopamine. It is also shown from the latter publication that 6,7-ADTN and derivatives thereof show dopamine-like activity in causing behavioral changes when administered cerebrally.

N-aralkyl derivatives are known both as dopamine compounds, such as (±)4-(2(3-p-hydroxyphenyl-1-methylpropyl)amino) ethyl-1-2-benzenediol (e.g. Dobutamine (Trade Mark: Lilly)) and as amino tetrahydronaphthalene compounds (see FR—A—2 373 513, claim 1, $R_5$ and page 16).

In accordance with the present invention there is provided the compound 2-$\alpha$-methyl-dopamin-imino-6,7-dihydroxyl-1,2,3,4-tetrahydronaphthalene, which has the formula

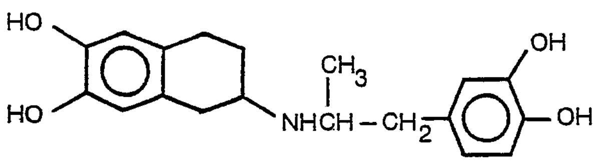

and pharmaceutically acceptable salts thereof.

The term "pharmaceutically acceptable salts" refer to acid addition salts prepared by reacting the basic amine of this invention with an organic or inorganic acid, or by reacting the amine salts with the salt of an appropriate acid. Representative salts which are so formed include the hydrochloride, hydrobromide, sulphate, bisulphate, acetate, oleate, laurate, benzoate, phosphate, citrate, maleate, succinate, tartrate and napsylate salts of the amines.

2-$\alpha$-methyl-dopaminimino-6,7-dihydroxy-1,2,3,4-tetrahydronaphthalene, may be prepared by reductive amination of protected (ADTN) which may be prepared by a modification of the preparation reported by J.G. CANNON et al. *(J. Med. Chem., 20,* p1111 (1977) and as illustrated in Scheme I below.

SCHEME I

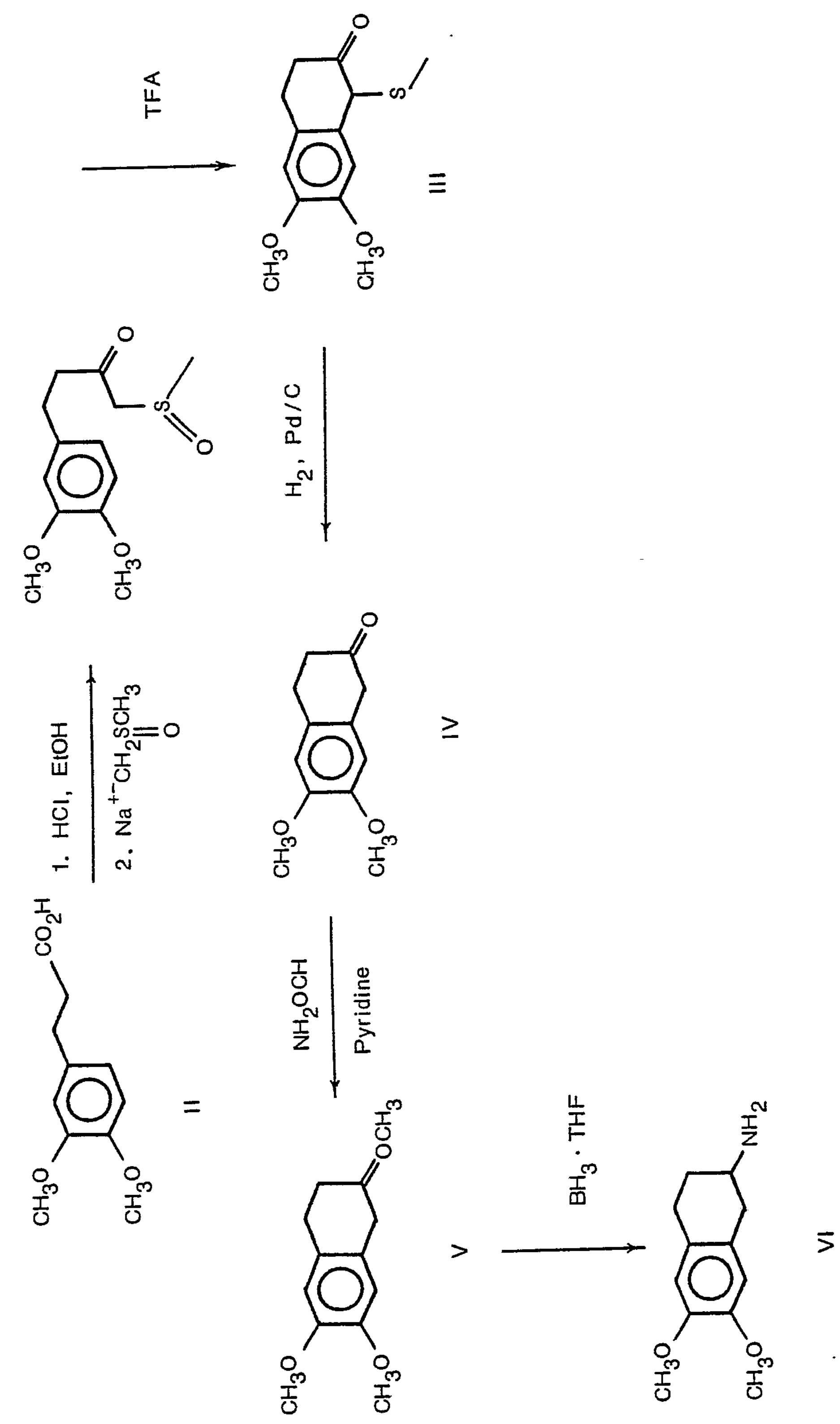

The preparation of 2-α-methyl-dopaminimino-6,7-dihydroxy-1,2,3,4-tetrahydronaphthalene by reductive amination of the protected (ADTN) is carried out with the appropriate ketone.

Generally, in the preparation of the 2-α-methyl-dopaminimino-6,7-dihydroxy-1,2,3,4-tetrahydronaphthalene, initially, a solution of 2-amino-6,7-dimethoxy-1,2,3,4-tetrahydronaphthalene (VI) in dry methanol is treated with the appropriate ketone, the hydroxyl groups of which are protected with methyl groups, and $NaCNBH_3$ under a nitrogen atmosphere. The mixture is then stirred for 72 hours whereupon concentrated HCl is added dropwise until the mixture becomes acidic. The methanol is removed on a rotary evaporator and the mixture is extracted with ether. The combined extracts are dried ($MgSO_4$) and the solvent is removed on a rotary evaporator. The products are dissolved in $CHCl_3$ and hydrogen chloride is bubbled in and the solvent removed on a rotary evaporator. The product is crystallized in isopropanol, the protecting groups are removed by a conventional method and the required compound is isolated.

The compounds of the present invention exhibit inotropic activity in warm-blooded animals at a dosage ranging from about 10.0 ug/kg to about 60.0 ug/kg of the body weight daily.

The following examples will further illustrate the present invention.

Example I

Preparation of 6,7-dimethoxy-2-methoxyamino-1,2,3,4-tetrahydronaphthalene (V)

A mixture of 0.56 g (6.7 mmol) of methoxylamine hydrochloride and 6.5 ml (6.5 mmol) of a 1*M* aqueous solution of NaOH was added to 0.689 g (3.34 mmol) of 6,7-dimethoxy-2-tetralone (*IV*). While under a nitrogen atmosphere the mixture was warmed to *ca.* 100° for 15 minutes and then kept at 40° using an infrared lamp for 18 hours. Water was added to the mixture which was then extracted with ether. The combined extracts were dried ($MgSO_4$) and the solvent was removed on a rotary evaporator, affording 0.700 g (89.2%) of brown oil, nmr ($CDCl_3$) 2.3—2.9 (m, 4H), 3.32 (S, 1H), 3.57 (S, 1H), 3.73 (S, 6H), 3.78 (S, 3H), 6.4—6.6 (m, 2H). The compound was used without further purification.

Example II

Preparation of 2-amino-6,7-dimethoxy-1,2,3,4-tetrahydronaphthalene (VI)

*2-amino-6,7-dimethoxy-1,2,3,4-tetrahydronaphthalene (VI).*

A solution of 0.700 g (2.98 mmol of *V* in 20 ml of dry THF was treated with 5.0 ml (5.0 mmol) of a 1*M* borane/THF solution while under a nitrogen atmosphere. The solution was heated to reflux for 6 hours and was then quenched with water. The aqueous mixture was extracted with $CHCl_3$, the combined extracts were dried and hydrogen chloride was bubbled into the solution. The solvent was removed on a rotary evaporator, leaving 0.775 g of off-white solid. Crystallization from isopropanol afforded 0.469 g (64.8%) of off-white product: mp. 83—86°C (lit. 85—87°C).

Example III

Preparation of 2-α-methyl-dopaminimino-6,7-dihydroxy-1,2,3,4-tetrahydronaphthalene, By Reductive Amination of Protected ADTN

A solution of 1 mmol of *VI* in 2 ml of dry methanol was treated with 1 mmol of the appropriate ketone, the hydroxyl groups of which were protected with methyl groups, and 1.05 mmol of $NaCNBH_3$ under a nitrogen atmosphere. The mixture was stirred for 72 hours whereupon concentrated HCl was added dropwise until the mixture became acidic. The methanol was removed on a rotary evaporator, the mixture was extracted with ether. The aqueous mixture was made basic (pH>12) with solid KOH and the mixture was extracted with ether. The combined extracts were dried ($MgSO_4$) and the solvent was removed on a rotary evaporator. The products were dissolved in $CHCl_3$, hydrogen chloride was bubbled in and the solvent was removed on a rotary evaporator. Crystallization of the products was carried out in isopropanol, the protecting methyl groups were removed and the required compound was isolated. The compound produced and the data of its preparation are as follows:— 2-α-methyl-dopaminimino-6,7-dihydroxy-1.2.3.4-tetrahydronaphthalene. This compound was isolated in 57% yield; nmr ($CD_3OD$) (1.32, d, J = 6 Hz, 3H), 1.5—4.0 (m, 10H) 6.4—6.8 (m, 5H).

The appropriate ketone referred to above is of the formula

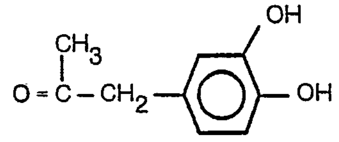

**Claims**

1. The compound of the formula

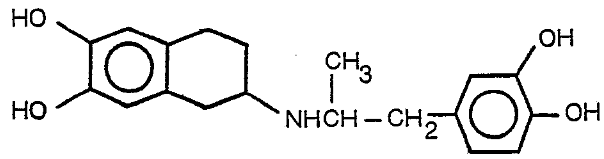

or a pharmaceutically acceptable salt thereof.

2. A process for preparing a compound according to claim 1, comprising reductive amination of 2-amino-6,7-dihydroxy-1,2,3,4-tetrahydronaphthalene, wherein the hydroxyl groups have been protected, with the ketone of formula

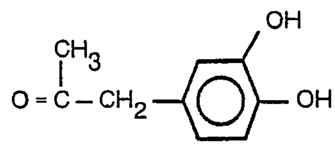

wherein the hydroxyl groups have also been protected and thereafter removing the protecting groups to obtain the compound of the formula given in claim 1 and optionally reacting said compound with an organic or inorganic acid.

**Revendications**

1. Composé de formule:

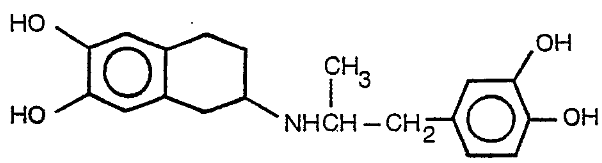

ou un sel pharmaceutiquement acceptable de ce composé.

2. Procédé de préparation d'un composé selon la revendication 1, comprenant l'amination réductrice de 2-amino-6,7-dihydroxy-1,2,3,4-tétrahydronaphtalène où les groupes hydroxyles ont été protégés, la cétone étant de formule:

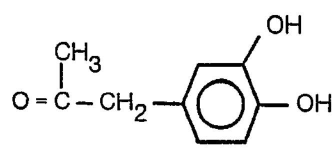

caractérisé en ce que les groupes hydroxyles ont été également protégés et en ce qu'on a ensuite retiré les groupes protecteurs pour obtenir le composé de la formule donnée à la revendication 1 en faisant éventuellement réagir ledit composé avec un acide organique ou inorganique.

**Patentansprüche**

1. Verbindung der Formel

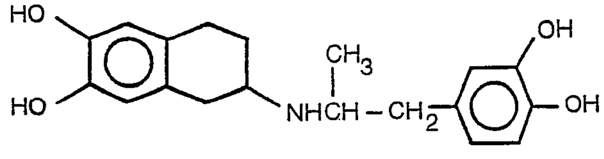

oder ein pharmazeutisch akzeptables Salz einer solchen Verbindung.

2. Verfahren zur Herstellung einer Verbindung nach Anspruch 1 durch reduktive Aminierung von 2-Amino-6,7-dihydroxy-1,2,3,4-tetrahydronaphthalin, dessen Hydroxylgruppen mit einem Keton der Formel geschützt sind

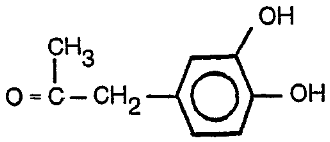

worin die Hydroxylgruppen ebenfalls geschützt sind, und anschließende Entfernung der Schutzgruppen zur Gewinnung der Verbindung der in Anspruch 1 angegebenen Formel, und gegebenenfalls Umsetzung dieser Verbindung mit einer organischen oder anorganischen Säure.